# EUROPEAN PATENT SPECIFICATION

(11) **EP 1 229 330 B1**
(45) Date of publication and mention of the grant of the patent: **21.02.2007**
(21) Application number: 00987766.3
(22) Date of filing: 27.12.2000
(51) Int. Cl.: G01N 33/68, C12N 15/09

(54) **PROBE FOR ANALYZING PROTEIN-PROTEIN INTERACTION AND METHOD OF ANALYZING PROTEIN-PROTEIN INTERACTIONS WITH THE USE OF THE SAME**
SONDE ZUM ANALYSIEREN VON PROTEIN-PROTEIN-WECHSELWIRKUNGEN UND ENTSPRECHENDES VERFAHREN DAFÜR
SONDE PERMETTANT D'ANALYSER UNE INTERACTION PROTÉINE/PROTÉINE ET MÉTHODE D'ANALYSE UTILISANT CETTE SONDE

(30) Priority: 26.07.2000 JP 2000224939
(43) Date of publication of application: 07.08.2002
(73) Proprietor: Japan Science and Technology Agency, Kawaguchi-shi, Saitama 332-0012 (JP)
(72) Inventor: UMEZAWA, Yoshio, Tokyo 162-0063 (JP); OZAWA, Takeaki, Matsudo-shi Chiba 270-2231 (JP)
(74) Representative: Dzieglewska, Hanna Eva
(86) International application number: PCT/JP2000/009348
(87) International publication number: WO 2002/008766

(56) References cited:
- EP-A- 0 602 899
- WO-A1-00/34514
- WO-A2-00/18881
- WO-A2-00/36093
- US-A- 5 795 731
- PERLER FRANCINE B ET AL: "Protein splicing elements: Inteins and exteins-a definition of terms and recommended nomenclature" NUCLEIC ACIDS RESEARCH, OXFORD UNIVERSITY PRESS, SURREY, GB, vol. 22, no. 7, 1994, pages 1125-1127, XP002154589 ISSN: 0305-1048
- SHINGLEDECKER, K. ET AL: "Molecular dissection of the Mycobacterium tuberculosis RecA intein: design of a minimal intein and of a trans-splicing system involving two intein fragments" GENE, no. 207, 1998, pages 187-195, XP004108918
- SOUTHWORTH M W ET AL: "Control of protein splicing by intein fragment reassembly" EMBO JOURNAL, OXFORD UNIVERSITY PRESS, SURREY, GB, vol. 17, no. 4, 1998, pages 918-926, XP002121550 ISSN: 0261-4189
- LEW B M ET AL: "Characteristics of protein splicing in trans mediated by a semisynthetic split intein" BIOPOLYMERS, NEW YORK, NY, US, vol. 51, no. 5, October 1999 (1999-10), pages 355-362, XP002162516 ISSN: 0006-3525
- OZAWA, T. ET AL: "A fluorescent indicator for detecting protein-protein interactions in vivo based on protein splicing" ANALYTICAL CHEMISTRY, vol. 72, no. 21, 1 November 2000 (2000-11-01), pages 5151-5157, XP001107084
- M.J. CLOSTON ET AL.: 'The ins and outs of protein splicing elements' MOLECULAR MICROBIOLOGY vol. 12, no. 3, 1994, pages 359 - 363, XP002937473

## Description

### TECHNICAL FIELD

The invention of this application relates to a probe for protein-protein interaction analysis, and to a method of using such probe for the analysis of protein-protein interactions. More precisely, the invention relates to a probe for protein-protein interaction analysis and to a method of using it for analysis of protein-protein interactions, which enables accurate and simple analysis of protein-protein interactions in all living cells.

### BACKGROUND ART

It is well known that protein-protein interactions play key roles in structural and functional organization of living cells.

Many unsolved problems currently studied in molecular biology and biochemistry, such as for gene transcription mechanism and intracellular information transmission are related to protein-protein interactions.

Some of the problems in the art of molecular biology and biochemistry have been gradually solved by the development of protein library screening techniques such as a two-hybrid strategy, in which a library of "target" proteins is screened for interaction with "bait" proteins (Chien, C. T., Bartel, P. L., Sternglanz, R., Fields, S., *Proc. Natl. Acad. Sci.* USA 1991, 88, 9578-9582; Fields, S., Song, O., *Nature 1989,* 340*,* 245-246). The two-hybrid method facilitates the identification of potential protein-protein interactions and has been proposed as an effective method for the generation of protein interaction maps (Flores, A., Briand, J. F., Gadal, O., Andrau, J. C., Rubbi, L., Mullem, V., Boschiero, C., Goussout, M., Marck, C., Carles, C., Thuriaus, P., Sentenac, A., Werner, M., *Proc. Natl. Acad. Sci. USA* 1999, *96*, 7815-7820; Ito, T., Tashiro, K., Muta, S., Ozawa, R., Chiba, T., Nishizawa, M., Yamamoto, K., Kuhara, S., Sakaki, Y., *Proc. Natl. Acad. Sci. USA* 1999, *97*, 1143-1147; Walhout, A. J. M., Sordella, R., Lu, X., Hartley, J. L., Temple, G. F., Brasch, M. A., Thierry-Mieg, H., Vidal, M., *Science* 2000, *287*, 116-122). However, the two-hybrid method has a problem in that it is applicable only to the detectable protein interactions that occur around the reporter gene in cell nuclei, but not to any other ordinary protein-protein interactions.

Another problem with the two-hybrid method is that its reliability is low. Therefore, every assay according to the method must be followed by an additional confirmation test using proteins of model cells or animals of which the functional data are known (Walhout, A. J. M., et al., *Science* 1999, *287,* 116-122).

Given that situation, other protein-protein interaction assay strategies have been developed. One is a ubiquitin split protein sensor (USPS) method which comprises reconstructing N- and C-terminal ubiquitins through protein-protein interaction, followed by activating the nuclear-localized reporter through transcription factor cleavage (Dunnwald, M., Varshavsky, A., Johnsson, *N., Mol. Biol. Cell* 1999, *10,* 329-344; Johnsson, N., Varshavsky, A., *Proc. Natl. Acad. Sci. USA* 1998, *95,* 5187-5192; Stagljar, I., Korostensky, C., Johnsson, N., Heesen, S., *Proc. Natl. Acad. Sci. USA* 1998, *95,* 5187-5192); another is an SOS recruit system, in which a catalyst domain is brought near to an intended membrane localization domain through protein-protein interaction thereby reconstructing a guanine exchange factor (GEF) or Ras, which then complements yeast causing temperature-sensitive mutation in yeast GEF (Aonheim, A., *Nucleic Acids Res.* 1997, *25,* 3373-3374; Aronheim, A., Zandi, E., Hennemann, H., Elledge, S. J., Karin, M., *Mol. Cell. Biol.* 1997, *17,* 3094-3102; Broder, Y. C., Katz, S., Aronheim, A., *Curr. Biol*. 1998, *8*, 1121-1124).

As a more general approach, split enzyme technology has been reported (Rossi, F., Charlton, C. A. and Blau, H. M., *Proc. Natl. Acad. Sci. USA* 1997, *94*, 8405-8410; Remy, I., Michnick, S. W., *Proc. Natl. Acad. Sci. USA* 1999, *96*, 5394-5399; Pelletier, J. N., Arndt, K. M., Pluckthun, A., Michnick, S. W., *Nature Biotech.* 1999, *17*, 683-690). It is said that, according to the method, a split enzyme is reconstructed through protein-protein interaction therein, and its enzymatic activity is restored. Further, it is said that the activity of the thus-reconstructed enzyme can be identified by its cellular phenotype or by its fluorescent substrate analogues.

These various methods produce accurate assay data in some degree and are well suited for assaying interactions between intracellular proteins and membrane-proximal proteins, however, they still contain problems in that they can be applicable to only appropriately engineered cells. Another such problem is that their accuracy is still unsatisfactory and their sensitivity is not sufficient. Still another problem is that such methods require different substrates and are therefore troublesome.

At present, a general method applicable to any protein that enables accurate and simple analysis of various protein-protein interactions, or an all-purpose probe for such method is not known.

The invention of the present application has been made in consideration of the above described situations, and its object is to solve the problems of the prior art and to provide an all-purpose probe applicable for various protein-protein interaction analysis and a method of protein-protein interaction analysis using such probe, which enables accurate and simple assay of the interaction of proteins.

### DISCLOSURE OF THE INVENTION

In order to solve the above-mentioned problems, the invention of this application firstly provides a probe for analyzing protein-protein interaction between two proteins, wherein said probe consists of two probes that are: probe a which comprises the N-terminal polypeptide of an intein and the N-terminal polypeptide of a labeled protein; and probe b which comprises the C-terminal polypeptide of the intein and the C-terminal polypeptide of the labeled protein.

Using such a probe, protein splicing is induced by protein-protein interaction, thereby regenerating a physiochemically or biochemically detectable protein.

Secondly, the invention provides the probe for protein-protein interaction analysis, wherein the C-terminal of probe a and the N-terminal of probe b each contain a linker sequence.

Thirdly, the present invention provides the probe for protein-protein interaction analysis, wherein the intein is an endonuclease derived from yeast VMA; and fourthly, the invention provides the probe for protein-protein interaction analysis, wherein the intein is DnaE derived from cyanobacterium.

Fifthly, the invention provides the probe for protein-protein interaction analysis, wherein the labeled protein is a fluorescent protein; and sixthly, the invention provides the probe for protein-protein interaction analysis, wherein the fluorescent protein is a green fluorescent protein (GFP)

Seventhly, the invention provides the probe for protein-protein interaction analysis, wherein the labeled protein is an emission-catalyzing enzyme; and eighthly, the invention provides the probe for protein-protein interaction analysis, wherein the emission-catalyzing enzyme is a luciferase.

Ninthly, the invention provides a method for analyzing protein-protein interaction, comprising: making a protein linked with probe a as described in claims 1 to 9 and a protein linked with probe b as described in claims 1 to 9 coexist in a system; and detecting the signal emitted by the labeled protein.

Tenthly, the invention provides the method of protein-protein interaction analysis, wherein a polynucleotide that expresses the probe of any one of claims 1 to 9 is introduced into a eukaryotic cell, thereby making the probe a-linked protein and the probe b-linked protein coexist in the cell.

### BRIEF DESCRIPTION OF THE DRAWINGS

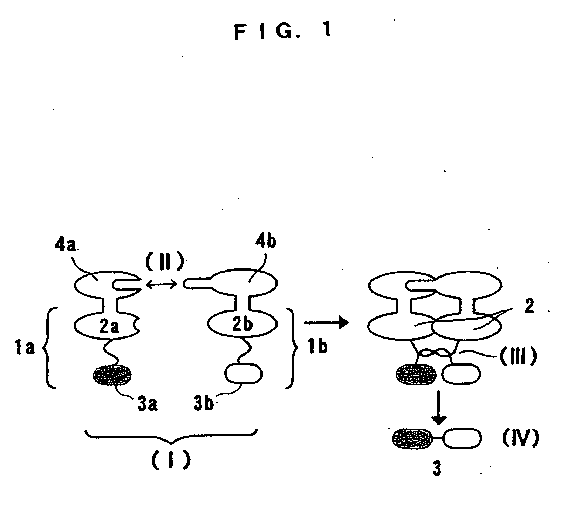

Fig. 1 shows an example of the structure and functional principle of the probe for protein-protein interaction analysis of the present invention. Here, (I) shows coexisting two probes; (II) shows protein-protein interaction; (III) shows protein splicing; and (IV) shows linkage of labeled protein. (1a) indicates probe a for protein-protein interaction analysis; (1b) indicates probe b for protein-protein interaction analysis; (2a) indicates the N-terminal polypeptide of an intein; (2b) indicates the C-terminal polypeptide of the intein; and (3) indicates a labeled protein. Further, (3a) indicates the N-terminal polypeptide of the labeled protein; and (3b) indicates the C-terminal polypeptide of the labeled protein. (4a) indicates protein (or a protein site) A; and (4b) indicates protein (or protein site) B.

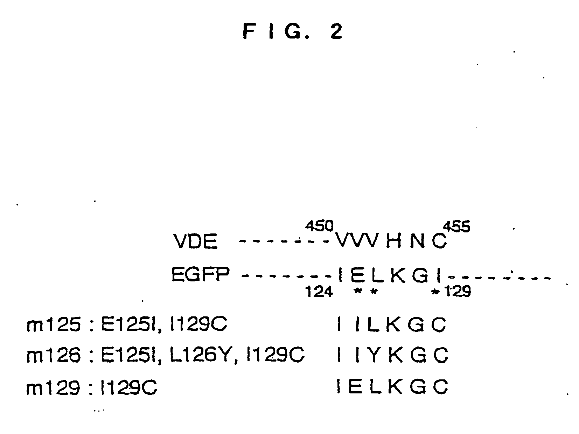

Fig. 2 shows the N-terminal polypeptide structure of EGFP in the Example of the present invention, for which the I124th to I129th amino acid residues have been replaced.

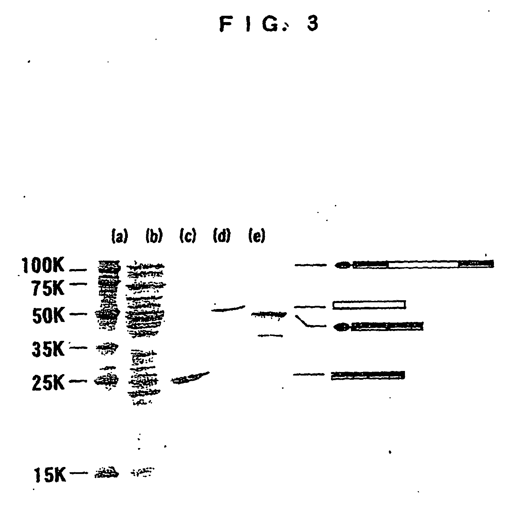

Fig. 3 shows the data of SDS-PAGE analysis of proteins expressed in *E. coli* transformed with pGEX-NVC. Here, lanes (a) to (c) are Coomassie Blue-stained SDS-PAGE; lane (a) indicates the protein molecular mass standard (Novagen) with their molecular masses (kDa); lane (b) indicates the crude extract before purification by GST-affinity column; and lane (c) indicates the sample purified by GST-affinity column. Lanes (d) and (e) show Western blotting analysis of crude lysate using antibodies specific to VDE (d) and to GFP (e).

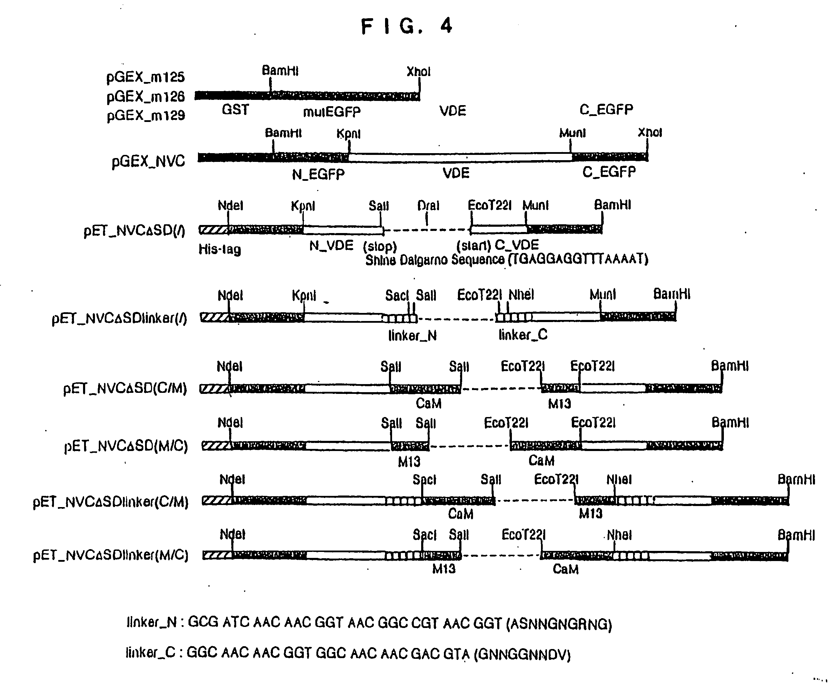

Fig. 4 shows the structure of a plasmid (probe <1> for protein-protein interaction analysis) constructed in the Example of the present invention. Here, the restriction endonuclease cleavage sites are shown above each bar; the dotted lines indicate the endonuclease domain in the intein VDE; the open bars indicate the intein VDE with no endonuclease; the black bars indicate GST labeling; and the shadowed bars indicate His labeling. The lattice patterns indicate a linker; "stop" indicates a translation termination codon; and "start" indicates a translation initiation codon.

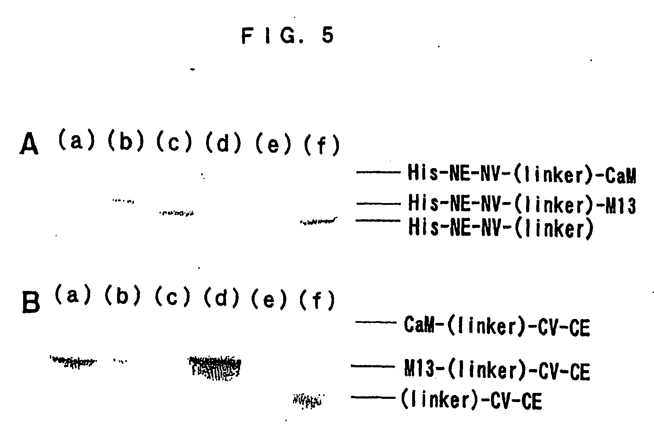

Fig. 5 shows the data of splicing induced by interaction of protein CaM and protein M13 in the Example of the present invention. Here, (a) indicates pET-NVCΔSD (C/M) (SDS-PAGE); (b) indicates pET-NVCΔSD (M/C); (c) indicates pET-NVCΔSD (/) gene 1; (d) indicates pET-NVCΔSD linker (C/M); (e) indicated pET-NVCΔSD linker (M/C); and (f) indicates pET-NVCΔSD linker (/). (A) shows the data of Western blotting labeled with anti-His; and (B) shows the data of Western blotting labeled with anti-GFP.

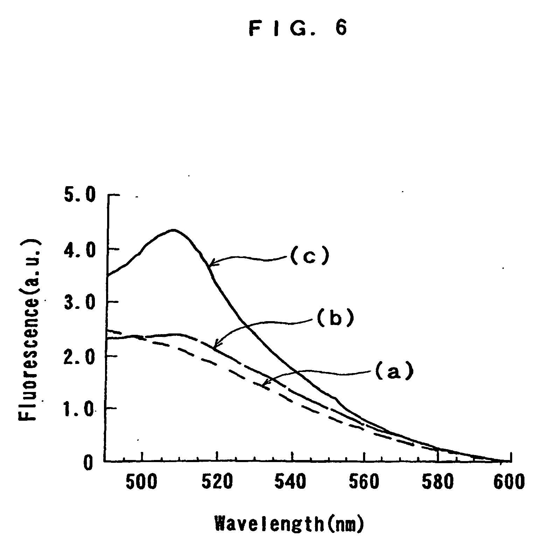

Fig. 6 shows the fluorescence spectra of crude products from *E. coli* carrying any of the plasmids (probe <1> for protein-protein interaction analysis) constructed in the Examples of the present invention. Excitation is at 470 nm, the excitation band width is 5.0 nm; and the emission band width is 5.0 nm. (a) indicates NVCΔSD linker (/) ; (b) indicates NVCΔSD (C/M); and (c) indicates NVCΔSD linker (C/M).

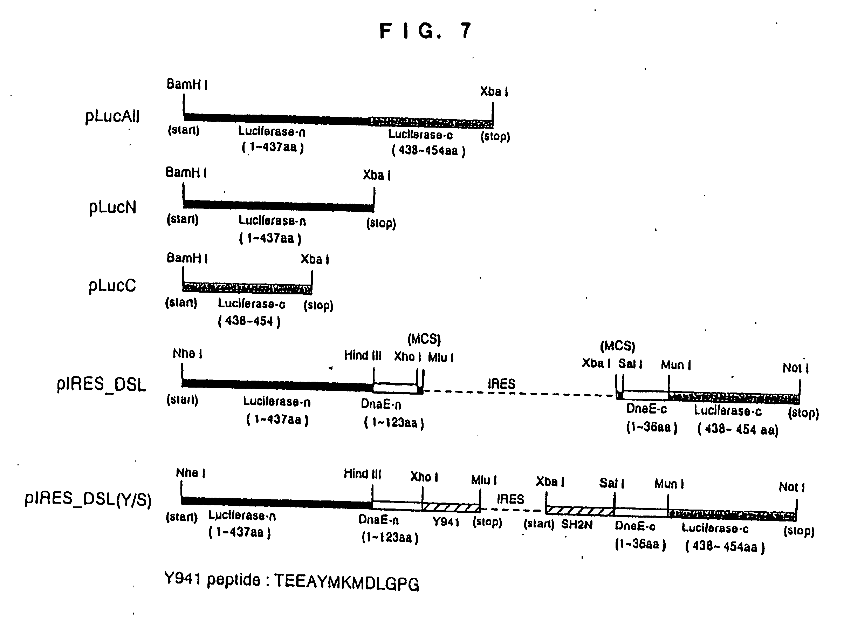

Fig. 7 shows the structure of a plasmid used in the Example of the present invention. Here, the dotted lines indicate an intracellular ribosome extein site (IRES) ; the cDNAs of pLucA11, pLucN and pLucC are inserted into pcDNA3.1 (+) ; "stop" and "start" indicate translation termination and initiation codons, respectively.

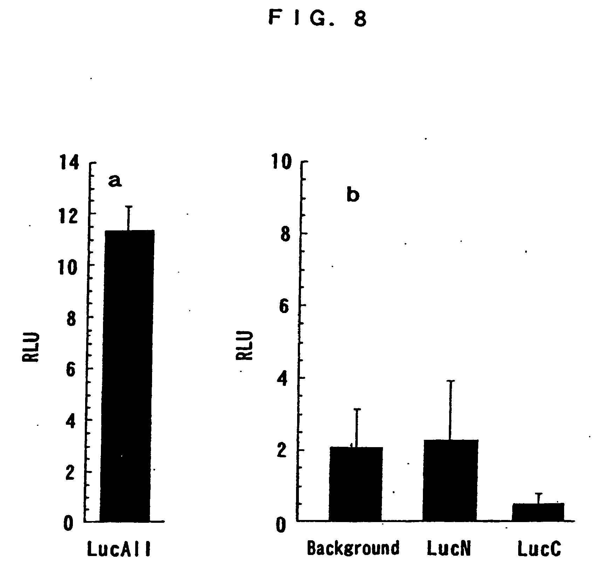

Fig. 8 shows the emission intensity of LucN alone, LucC alone and LucAll in the Example of this invention.

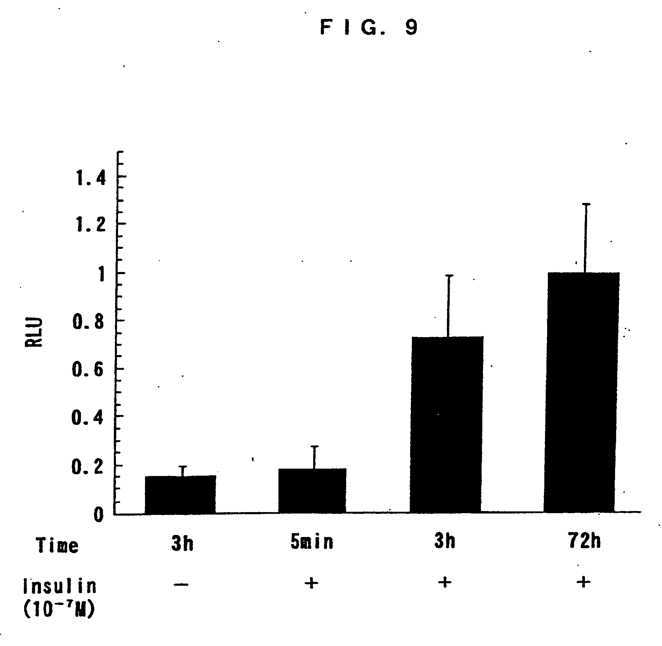

Fig. 9 shows the insulin-dependent protein splicing increase in the Example of this invention.

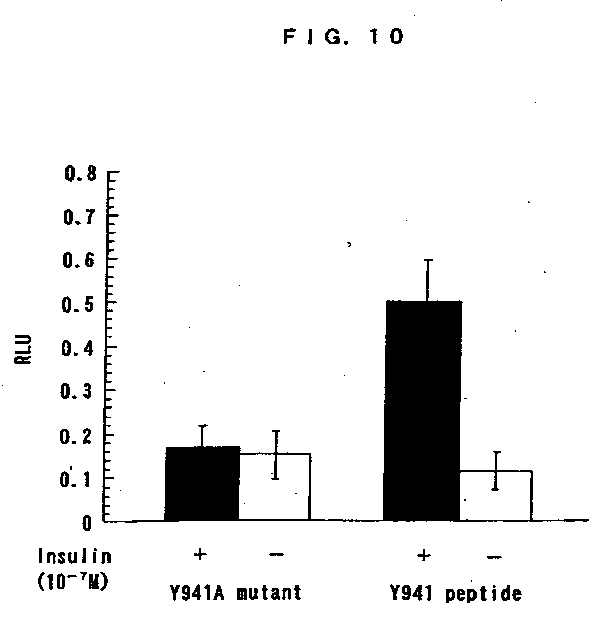

Fig. 10 shows the influence of amino acid mutation on protein splicing in the Example of this invention.

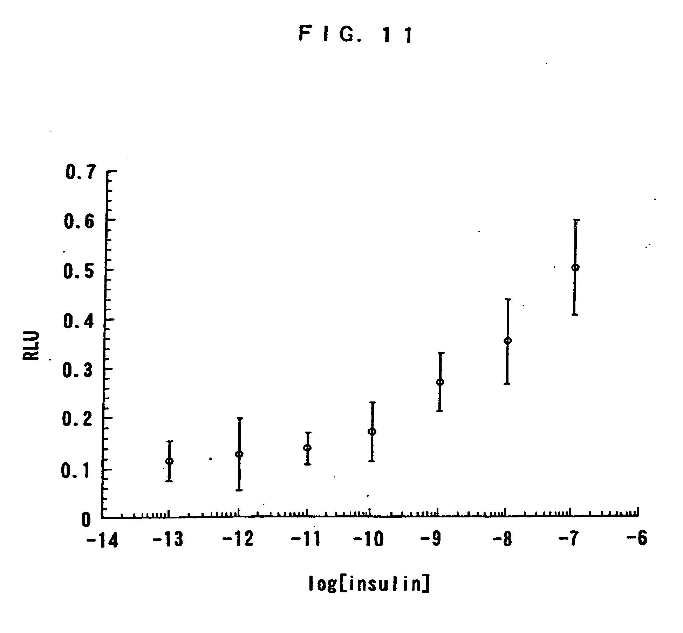

Fig. 11 shows the insulin concentration dependency of Lum-F emission intensity in the Example of this invention.

### BEST MODE FOR CARRYING OUT THE INVENTION

The probe for protein-protein interaction analysis of the present invention is based on the principle that the interaction of two proteins each linked to the probes induces splicing thereby regenerating the labeled protein split and attached to the two probes, to emit a signal.

Protein splicing is a process wherein the internal protein segment (intein) is excised from a translated protein. In this process, the excision of inteins is accompanied by the ligation of flanking sequences (exteins) (Gimble, F. S., *Sci. Biol.* 1998, *5*, R251-256).

The probe for protein-protein interaction analysis of the present invention is provided by ingeniously applying the self-excision of inteins to protein-protein interaction analysis.

In other words, the probe for protein-protein interaction analysis of the present invention consist of two probes a and b each separately connected to two different proteins. Fig. 1 is a schematic representation of the principle of the probe for protein-protein interaction analysis of the present invention.

Of the two probes a (1a) and b (1b), probe a (1a) contains the N-terminal polypeptide of an intein (2a) and the N-terminal polypeptide of a labeled protein (3a); and probe b (1b) contains the C-terminal polypeptide of the intein (2b) and the C-terminal polypeptide of the labeled protein (3b).

The two probes a (1a) and b (1b) are separately connected to the respective proteins A (4a) and B (4b) for which interaction is to be analyzed, to detect the protein A-protein B interaction. Hence, when protein A (4a) and protein B (4b) each connected to probe a (1a) and probe b (1b), respectively, coexist (I) and when the two proteins (4a, 4b) interact with each other (II), the intein (2) is excised through splicing (III) . As a result, the labeled protein sites (3a, 3b) bonded to the intein (2) are ligated (IV), thereby enabling the confirmation of the protein-protein interaction through detection of the signal resulting from the labeled protein (3).

In this process, if protein A (4a) does not interact with proteinB(4b), splicingof intein (III) does not occur; therefore, the labeled protein (3) is not regenerated (IV) and is not detectable.

For the probe for protein-protein interaction analysis of the present invention, probes a and b (1a and 1b) may each be composed of the intein polypeptide and the labeled protein polypeptide, but may contain other parts such as a linker sequence. Accordingly, in probes a and b (1a and 1b), the intein polypeptides (2a, 2b) and the labeled protein polypeptides (3a, 3b) may be directly bonded to each other, or may be bonded via any other polypeptide such as linker sequence.

The intein may be derived from various known organisms. It includes, for example, those derived from eucaryotes such as *Saccharomyces cerevisiae* (yeast) Sce VMA, *Candida tropiallis* (*Candida* fungi) Ctr VMA; those derived from eubacteria such as *Mycobacterium tuberculosis* (tubercle bacillus) Mtu recA; and those derived from paleobacteria such as *Thermoplasma asidophilum* Tac VMA*.* Inaddition, *Cyanobacterium synechocystis sp*. (cyanobacterium) DnaE may also serve as the intein for use in the invention.

When protein A (4a) interacts with protein B (4b) in the presence of the probes for protein-protein interaction analysis (1a, 1b) of the present invention, the inteins are preferably site-specific endonucleases for facilitating automatic intein excision.

Specifically, intein derived from yeast VMA and Ssp DnaE intein derived from cyanobacterium are preferable. In yeast VMA, the nascent translation product, 120-kDa VMA1, catalyzes protein splicing to give a 70-kDa H*-ATPase sub-unit and a 50-kDa site-specific endonuclease (VDE, or PI-SceI). This VDE is preferably used as the intein sites (2a, 2b) in the probes for protein-protein interaction analysis (1a, 1b). For the Ssp DnaE derived from cyanobacterium, the DNA sequence of the strain PCC6803 has been determined (the N-terminal has 123 amino acid residues and the C-terminal has 36 amino acid residues); further, Ssp DnaE intein is a natural split intein, and is known that the ligation of N- and C-extein occurs (Wu, H., Hu, Z., Liu, X. *-*Q., *Proc. Natl. Acad. Sci. USA* 1998*, 95*, 9226-9231). Therefore Ssp DnaE intein is easy to handle, and preferred as the intein sites (2a, 2b) in the present probes for protein-protein interaction analysis (1a, 1b).

Of the various inteins mentioned above, the cyanobacterium-derived Ssp DnaE intein is most preferable for enabling highly sensitive detection of protein-protein interactions in mammal cells. It is needless to say, that any other known or novel inteins may alsobe employed in the invention.

For effective intein splicing (III) with the probes for protein-protein interaction analysis (1a, 1b) of the present invention, probes 1a and 1b must be correctly folded so that the two sites participating in protein splicing are adjacent to each other and are correctly aligned (Duan, X., Gimble, F. S. and Quiocho, F. A., *Cell* 1997*, 89,* 555-564). Accordingly, as the inteins, those derived from organisms may directly be used or may be suitably modified for facilitating the intended intein splicing by, for example, partly substituting the amino acid residues of the organisms-derived intein with any other residue, partly deleting amino acid residues, or introducing a suitable linker sequence thereinto.

For example, for the above-mentioned VDE, it is known that its mutant specifically designed by deletion of the endonuclease domain and substitution with a foldable dodecapeptide linker shows an increased splicing activity (Cooper, A. A., Chen, Y. J., Lindorfer. M. A., Stevens, T. H., *EMBO J.* 1993, 12, 2575-2583; Chong, S., Xu, M. -Q., *J. Biol. Chem.* 1997, *272,* 15587-15590). On the other hand, the cyanobacterium-derived Ssp DnaE is a natural split intein, as mentioned above, and is known to lead to correctly folded splicing. Therefore, linker sequence may or may not be introduced into it.

As described hereinabove, the intein is split into the N-terminal polypeptide (2a) and the C-terminal polypeptide (2b). In the probes (1a, 1b) for protein-protein interaction analysis of the present invention, the split polypeptides (2a, 2b) are bonded to the N-terminal polypeptide (3a) of a labeled protein and the C-terminal polypeptide (3b) thereof, respectively, to construct a probe.

On the other hand, for the probes for protein-protein interaction analysis (1a, 1b) of the present invention, the labeled protein sites to be used undergo direct peptide bonding and link to each other (IV) through the interaction of proteins A an B (II), followed by the intein splicing from probes a and b (1a, 1b) (III). The labeled protein (3) may be any protein that can be detected after protein linkage (IV). For example, fluorescent protein and emission-catalyzing enzyme are preferable. As a fluorescent protein, green fluorescent protein (GFP), which emits light after protein linkage, enabling visual detection, is preferable. For the emission- catalyzing enzyme, luciferase that forms an activity center after protein linkage and emits light that is easily detected is preferred. For the split N- and C-terminals of the luciferase to give no fluorescence when separated and restore its emission activity after its two terminals are recombined, the luciferase must be split so that its activity center is divided into two. It is known that the enzyme luciferase is folded into two domains that sandwich a broad activity center-containing region therebetween ; one is a large N-terminal domain comprising one β-barrel and two β-sheets and the other is a C-terminal site (Waud, J. P., Sala-Newby, G. B., Matthews, S. B., Campbell, A. *K., Biochim. Biophys. Acta* 1996, *1292*, 89-98; Conti. E. Franks, N. P., Brick, P., *Structure* 1996, *4*, 287-298). Accordingly, it is desirable that the enzyme luciferase is split into two, 3a and 3b, at the flexible side through which the two domains are linked to each other. For the enzyme luciferase, it is further known that its N-terminal polypeptide (3a), to which the N-terminal polypeptide of the intein (2a) is to be bonded, has a cysteine (Cys) residue and further has a tyrosine (Tyr) residue -1 upstream to it and alanine (Ala) and phenylalanine (Phe) residues -3 and -4 upstream to it, respectively; therefore, the enzyme luciferase enables more efficient protein splicing. Hence, for using the enzyme luciferase in the present invention, it is recommended that the enzyme be mutated into mutants R437C for which the 437th arginine is substituted with cysteine, D436Y for which the 436th aspartic residue is substituted with tyrosine, and I434A for which the 434th isoleucine residue is substituted with alanine, to enable a more efficient protein splicing.

As mentioned hereinabove, the invention of this application provides the probe for protein-protein interaction analysis. Concretely, one probe (e.g., probe a) is linked to the targetprotein (protein A) of which theabilityof interaction with another protein (protein B) is tobe confirmed, whileanother probe (probe b) is linked to the other protein (protein B) ; and the two probes are made to coexist in a system to thereby confirm the presence or absence of interaction between proteins A and B by the above-mentioned principle and mechanism.

The proteins (4a, 4b) may be linked with the probes (1a, 1b) by any method as long as the properties of the proteins and the probes are not affected. For example, any ordinary chemical, biochemical or genetic engineering strategies are applicable.

The method of using the probe for protein-protein interaction analysis of the invention for detecting and analyzing protein-protein interactions (e.g., through luminometry) is not limited, and may be done by any ordinary means. Experimental methods and detectors generally used in the field of chemistry and biochemistry may be employed. For example, a luminometer, which enable simple detection and analysis is preferable.

Embodiments of the invention are described in more detail in the following Examples with reference to the drawings attached hereto. It is needless to say, that the invention is not limited to these Examples, and various changes and modifications may be made without limitation.

### EXAMPLES

### Example 1: Construction of Probe <1> for protein-protein interaction analysis

As the intein site of probe <1> for protein-protein interaction analysis, a yeast VMA1-derived intein (VDE) was used.

Of the 454 amino acids constituting VDE, Cys1 is the first amino acid residue and Asn454 is the last amino acid residue. For VDE, the C-extein starts from Cys455, and the extein residue adjacent to Cys1 is numbered -1. The subsequent extein residues are numbered -2, -3, ...consecutively toward the N-extein.

For the labeled site, an Enhanced Green Fluorescent Protein derived from *Aequoea victoria* (EGFP, described, for example, in *Current Biology* 1996, 6 (2), 178-182) was used.

In probe <1>, as the splicing site, the N-terminal half of EGFP requires one Cys residue, another Gly residue upstream of the position of -1, and three hydrophobic amino acid residues at their positions of -5, -4 and -3.

Accordingly, the I124th to I129th amino acid residues that are relatively stable in the N-terminal polypeptide structure of EGFP were substituted as follows (Fig. 2).
(1) I129C and E125I mutation in EGFP showed fluorescence, and excitation and emission peaks equivalent to those of EGFP at 488 nm and 510 nm were observed.
(2) Fluorescence disappeared with L126Y mutation at m125, although the expression level of the mutant did not vary.

The results indicate that L126Y mutation disables correct folding in the m129EGFP mutant and/or ligation of the fluorescent protein sites could not take place.

Accordingly, m125EGFP mutant for which I129C and E125I mutation was performed was used as the labeled protein site of the probe. This labeled protein site is hereinafter referred to as an EGFP mutant.

### Example 2: Confirmation of Splicing in single polypeptide

To confirm whether protein splicing occurs in a single polypeptide in which VDE is sandwiched between the N-terminal and the C-terminal halves of the EGFP mutant, pGEX-NVC was expressed at 25°C in *E. coli.*
(1) Cells of E. coli DH5α were incubated to express a glutathion S-transferase fused protein(GST). A plasmid that covers the VDE region and the N- and C-terminal polypeptides of the EGFP mutant was fused to the GST gene under control or a tac promoter. This produced a chimera protein consisting of GST (26 kDa), 125 residues from the N-terminal of the EGFP mutant (13 kDa), VDE (50 kDa) and the C-terminal polypeptide of the mutant (14 kDa).
(2) The resulting protein was extracted from the *E. coli* cells, purified and identified through SDS-PAGE. The labeled protein and 10 to 225-kDa (Novagen) were electrophoresed on 12 to 15 % SDS-PAGE gel. The gel was visualized by Coomassie Brilliant Blue staining.
   For Western blotting, anti-VDE polyclonal antibody, anti-His-labeled polyclonal antibody (Santa Crus Biotechnology) or anti-GFP monoclonal antibody (BioRad) was used as the probe.
   The enzymes necessary for cloning were all obtained from Takara Biomedical, and processed according to the manufacturer's instructions.
   The PCR fragments were sequenced using a genetic analyzer, ABI310.
   The main component of the crude product was a protein of about 50-kDa (Fig. 3b). This result corresponds with the size of the extein linked to VDE (50 kDa) and GST. In other words, this component was determined to be a fused protein of GST and the N-terminal polypeptide of the EGFP mutant (26 kDa + 13 kDa). From this result, it was found that the 103-kDa precursor of the fused protein was split into the 50-kDa VDE and the 53-kDa GST-EGFP mutant fused protein.
   In addition, the molecular weight of VDE and that of the GST-EGFP mutant fused protein were measured through Western blotting.
   The anti-VDE and anti-GFP antibodies specifically reacted with the excised 50-kDa intein (Fig. 3d) and the 53-kDa GST-EGFP mutant fused protein (Fig. 3e), respectively. All components of about 100 kDa of the unspliced precursor were analyzed using these antibodies.
   The GST-EGFP mutant fused protein was further identified by GST-affinity chromatography. The crude extract was passed through an affinity column, and the proteins bound to the resin were extracted using PreScission protease and then subjected to SDS-PAGE (Fig. 3c). This gave a 25-kDa band, which was almost the same as the molecular weight of the EGFP mutant.
(3) Next, the affinity column-purified protein was subjected to fluorescent spectrometry, for which the excitation and emission peaks appeared at 488 nm and 510 nm, respectively. The data well corresponded to those of EGFP.

The above results indicated that the VDE in the center of the single polypeptide was excised by splicing, causing ligation of the N- and C-terminal polypeptides of the EGFP mutant by peptide bonding, resulting in the correct folding of EGFP mutant to form a fluorophore.

### Example 3: Effect of Probe <1> for protein-protein interaction analysis

(1) Cells of *E. coli* BL21(DE3)pLysS were incubated to produce a recombinant fused protein labeled with His at its N-terminal.
   In order to test Protein-protein interaction, GST of pGEX-NVC was substituted with His-label of a pET16-b vector, and its splicing functional site was split to obtain pET-NVCΔSD(/).
   The splitting was realized by substituting the nonfunctional endonuclease motif that exists in the 185th to 389th amino acid region of the mutant with a cassette of ((translation termination codon) - (Shine-Dalgarno sequence) - (translation initiation codon)).
   The resultingplasmidpET-NVCASD(/) was a two-gene operon essentially composed of gene 1 that encodes the N-terminal polypeptide of EGFP and VDE (N-EGFP-VDE) and gene 2 that encodes the C-terminal polypeptide of VDE and EGFP (C-VDE-EGFP).
   For the N- and C-terminal polypeptides of VDE to be near each other in space during protein-protein interaction, a foldable peptide linker having repetitive Gly-Asn sequences was introduced between the translation termination codon and the translation initiation codon (pet-NVCΔSD linker (/)).
   In order to confirm whether specific protein-protein interaction that facilitates the splicing to give EGFP in *E. coli* occurs, calmodulin (CaM) and its target peptide M13 were selected as model proteins.
   The structure of CaM and that of M13 have been clarified through NMR (Ikura, M., et al., *Science* 1992, *256,* 632-638); therefore, CaM and M13 are suitable because the distance between any amino acid in CaM and any amino acid in M13 is known.
(2) The recombinant plasmids pET-NVCΔSD(C/M), pET-NVCΔSD linker (C/M) and pET-NVCΔSD linker (/) were introduced into the cells of *E. coli* to obtain the corresponding fused protein.

For intracellular splicing, the protein expression was effected at 25°C for 12 hours; the cells were stored at 4°C for 1 or 2 days.

Gene 1 protein expression was confirmed by an anti-His labeled antibody (Fig. 5A). The major components analyzed by the antibody were proteins of 55 kDa (Fig. 5A a, d), and their size is on the same level as that of the unspliced N-EGFP-VDE precursor (36 kDa) with CAM (17 kDa) or its linker (1 kDa).

As the control, a lysate of E. coli having a pET-NVCΔSD linker (/) plasmid was subjected to SDS-PAGE, which gave a single band for N-EGFP-VDE (Fig. 5A, f).

The expression level of the unspliced precursor proteins obtained from these three plasmids was almost the same.

On the other hand, the gene 2 protein product was identified by an anti-GFP monoclonal antibody, and was found to be the C-terminal polypeptide of EGFP.

In addition, in the cell that expressed pET-NVCΔSD(C/M) and pET-NVCΔSD linker (C/M), the size of the expression protein obtained from the gene operon well corresponded with that of the precursor protein of N-VDE-EGFP (20 kDa) with M13 (3 kDa) (Fig. 5B, a) or the linker-containing M13 (4 kDa) (Fig. 5B, d) . Similarly, the control plasmid expressed the expected protein (Fig. 5B, f). The expression levels of all three proteins obtained from operon II were the same.

Fig. 6 shows the fluorescent spectra of lysates of *E. coli* cells each carrying one of the above-mentioned plasmids. For the cells of *E. coli* carrying pET-NVCΔSD linker (/) no spectral change was observed. From the cells of *E. coli* carrying pET-NVCΔSD(C/M) and having co-expressed CaM and M13, a slight change in fluorescence emission at 510 nm was observed. On the other hand, from the cells of *E. coli* carrying N-EGFP-VDE and C-VDE-EGFP with a peptide linker therebetween and having co-expressed CaM and M13, a significant change in the fluorescence emission was observed at 510 nm. The fluorescence intensity of the crude product from the cells of *E*. *coli* that carries pET-NVCΔSD linker (C/M) wasenoughtodifferentiatethose cells from the cells that carry the control plasmid, pET-NVCΔSD linker (/) or the plasmid not encoding the foldable linker pET-NVCΔSD linker (C/M).

These results indicate that the CaM-M13 interaction resulted in in-trans splicing, thereby inducing the ligation of the two external regions of N- and C-terminal polypeptides of the EGFP mutant to form the EGFP fluorophore.

### Example 4: Effect of Linker in Probe for protein-protein interaction analysis <1>

For protein splicing, it is necessary that the N- and C-terminals of VDE be correctly folded. Correct folding is attained only when the C-terminal N-VDE is close to the N-terminal C-VDE.

Regarding CaM and M13, the distance between the N-terminal of CaM and the C-terminal of M13 is 50 Å (from Brookhaven Protein Data Bank). This distance is too large for N-VDE to be close to C-VDE, and the large distance was expected to interfere with the correct folding of the N- and C-terminals of VDE in E. coli carrying the plasmid pET-NVCΔSD linker (C/M).

However, the plasmid pET-NVCΔSD linker (C/M) actually increased the fluorescence intensity at 510 nm significantly. This result confirms that the 10- and 9-amino acid linkers each linked to the N- and C-VDEs enabled the necessary foldability of VDE and realized good conformation for effective VDE splicing.

### Example 5 : Construction of Probe for protein-protein interaction analysis <2>

This experiment was performed to demonstrate the construction of a plasmid in a host, *E. coli* DH5α.

As the intein site for a probe <2>, DnaE derived from cyanobacterium, *Synechocystis sp.* PCC6803 was chosen. As the labeled protein for the probe, a wild firefly-derived luciferase (Lum-F) (pLucAll) was split between the 437th amino acid and the 438th amino acid into N-terminal (pLucN) and C-terminal (pLucC) segments.

Fig. 7 shows the structure of the luciferase and luciferase segments.

### (1) Splitting of Luciferase:

First, the absence of enzymatic activity inpLucN and pLucC was confirmed by transitionally expressing the two luciferase segments in human insulin receptor-overexpressing Chinese hamster ovarian cells (CHO-HIR) .

Concretely, the CHO-HIR cells were transfected with 1 µg of each plasmid (pLucAll, pLucN, and pLucC) or with 0.01 µg of a control plasmid (pRL-TK), and incubated in a 12-well plate for 45 hours. After incubation, the emission of the cells in each well was measured using a luminometer.

As a blank control, the CHO-HIR cells with no plasmid were incubated under the same condition.

The transfection efficiency error in each well of the plate was corrected through dual-luciferase assay using a *Renilla*-derived luciferase (lum-R).

Fig. 8 shows the relative emission intensity (RLU) of each luciferase (or each luciferase segment).

The RLU of the CHO-HIR cells with the wild firefly luciferase (LucAll) expressed was 11.4 (Fig. 8a); however, the RLU of the CHO-HIR cells with only LucN or LucC expressed was 2.2 × 10⁻⁴ and 4.5 × 10⁻⁵, respectively (Fig. 8b). The RLU (background) of the CHO-HIR cells transfected with Lum-R itself was 2.2 × 10⁻⁴. This result indicates that LucN or LucC alone shows no emission activity.

### (2) Splitting of DnaE:

As mentioned above, *Synechocystis sp.* PCC6803-derived DnaE was split into an N-terminal segment of 123 amino acid residues and a C-terminal segment of 36 amino acid residues.

### (3) Probe <2>:

The N-terminal segment of DnaE in (2) was ligated with LucN in (1) while the C-terminal segment of DnaE in (2) was ligated withLucCin (1) . These segments were inserted into a bicistronic expression vector, pIRES (Invitrogen) at the multicloning site (MCS) to construct pIRES-DSL. The resulting plasmid pIRES-DSL had two MCSs at the 3'-terminal of the N-terminal DnaE and at the 5'-terminal of the C-terminal DnaE (MCS-A and MCS-B, respectively), to which proteins or protein domains which may interact with each other or which are to be tested for their interactivity are introduced.

Fig. 7 shows the structure of pIRES-DSL.

### Example 6: Effect of Probe <2> for protein-protein interaction analysis 1

(1) This experiment was performed to confirm whether probe <2> constructed in Example 5 is effective for luciferase splicing, using an oligopeptide (Y941) that contains the 941st tyrosine residue of IRS-1 (which is known to participate in insulin signal transduction) and its target protein, phosphatidylinositol 3-kinase-derived SH2N domain (White, M. F., *Diabetologia* 1997, *40*, S2-S17).

A plasmid pIRES-DSL (Y/S) with Y941 inserted into MCS-A and SH2N into MCS-B, and a pRL-TK vector were transiently coexpressed in CHO-HIR cells.

Fig. 7 shows the structure of the pIRES-DSL (Y/S).

First, in a 6-well plate, CHO-HIR cells were transfected with 2 µg of the plasmid pIRES-DSL (Y/S) or 0.02 ng of the control plasmid pRL-TK. The cells were incubated for 45 hours, and then the culture was substituted with an FBS-free solution of 1.0 × 10⁻⁷ M human insulin; the CHO-HIR cells were excited through incubation (at 37°C) for 72 hours, 3 hours or 5 minutes. The cells incubated for 5 minutes were further incubated for 175 minutes in an FCS-free culture with no insulin therein.

Fig. 9 shows the relative emission intensity, RLU, of the insulin-excited cells.

The corrected luciferase activity of the CHO-HIR cells excited with insulin for 72 hours, 3 hours and 5 minutes were 1.0, 0.73, and 0.18, respectively. The relative emission intensity (against background) of the cells incubated in the insulin-free culture was 0.15, and this is nearly the same as that of the cells excited with insulin for 5 minutes.

In addition, it is understood that the relative emission intensity, RLU of the cells excited with insulin for 3 hours or longer is more than 4 times that of the background. The kinase activity of the cells tested herein results from the specific interaction between the peptide Y941 (the 941 amino acid of which is tyrosine) phosphorylated with an insulin receptor, and SH2N. This enabled the efficient in-trans folding and splicing of DnaE in probe <2>, thereby causing the ligation of LucN and LucC to realize luciferase emission reproduction.

Next, an Y941 mutant (wherein the tyrosine residue in Y941 was substituted with an alanine residue that is not phosphorylated with the insulin receptor) was constructed, and tested in the same manner as described above.

The results are shown in Fig. 10.

The corrected luciferase emission of the Y941 mutant-expressing CHO-HIR cells was on the same level as that of the background data. This confirms that the tyrosine phosphorylation in Y941 led to the protein-protein interaction between Y941 and SH2N.

### Example 7: Effect of Probe <2> 2

Probe <2> constructed in Example 5 was tested for quantitative analysis of insulin-induced protein-protein interaction in CHO-HIR cells.

First the insulin concentration dependence of the RLU was confirmed by the following procedure: pIRES-DSL (Y/S) and pRL-TK were coexpressed in the CHO-HIR cells in the same manner as above, and the cells were then excited with insulin of various concentrations between 1.0 × 10⁻¹³ and 1.0 × 10⁻⁷ M, at 37°C for 3 hours.

Fig. 11 shows the RLU of the cells.

The results indicate that the emission intensity of the cells increases with insulin concentration.

### INDUSTRIAL APPLICABILITY

As described in detail hereinabove, the present invention provides a probe for protein-protein interaction analysis that enables accurate and simple analysis of protein-protein interactions. The invention also provides a method for the analysis of protein-protein interactions using the probe.

The present method for analysis of protein-protein interactions does not require any reporter gene or substrate as in conventional methods, and enables simple and accurate analysis of protein-protein interactions. The method and the probe are applicable even to mammal cells for high-sensitivity analysis of protein-protein interactions in the cells.

Accordingly, the probe for protein-protein interaction analysis of the present invention enables analysis of the mechanisms of protein-protein interactions in various organisms, such as protein-protein interactions in cell membranes and receptor activation by hormones in cells, in a simplified manner that requires little time.

## Claims

1. A probe for analyzing protein-protein interaction between two proteins, consisting of two probes that are: probe (a) which comprises the N-terminal polypeptide of an intein and the N-terminal polypeptide of a labelled protein; and probe (b) which comprises the C-terminal polypeptide of the intein and the C-terminal polypeptide of the labelled protein.

2. The probe for protein-protein interaction analysis of claim 1, wherein the C-terminal of probe (a) and the N-terminal of probe (b) each contain a linker sequence.

3. The probe for protein-protein interaction analysis of claim 1 or 2, wherein the intein is an endonuclease derived from yeast VMA.

4. The probe for protein-protein interaction analysis of claim 1 or 2, wherein the intein is DnaE derived from cyanobacterium, Ctr VMA derived from Candida fungi, Mtu recA derived from Mycobacterium tuberculosis or Tac VMA derived from Thermoplasma asidophilum.

5. The probe for protein-protein interaction analysis of claims 1 to 4, wherein the labelled protein is a fluorescent protein.

6. The probe for protein-protein interaction analysis of claim 5, wherein the fluorescent protein is a green fluorescent protein.

7. The probe for protein-protein interaction analysis of claims 1 to 4, wherein the labelled protein is a luminescent enzyme.

8. The probe for protein-protein interaction analysis of claim 7, wherein the luminescent enzyme is a luciferase.

9. The probe of claim 8, wherein the luciferase has the following mutations: R437C, D436Y and I434A.

10. A polynucleotide encoding the probe of any one of claims 1 to 9.

11. A vector containing the polynucleotide of claim 10.

12. A eukaryotic cell containing the polynucleotide of claim 10 or the vector of claim 11.

13. A method for analyzing protein-protein interaction, comprising:
making a protein linked with a probe as described in claims 1 to 9 and a protein linked with probe b as described in claims 1 to 9 coexist in a system; and
detecting the signal emitted by the labelled protein.

14. The method of protein-protein interaction analysis of claim 13, wherein a polynucleotide that expresses the probe of any one of claims 1 to 9 is introduced into a eukaryotic cell, thereby making the probe a-linked protein and the probe b-linked protein coexist in the cell.

## Patentansprüche

1. Sonde zur Analyse von Protein-Protein-Wechselwirkung zwischen zwei Proteinen, bestehend aus zwei Sonden, bei denen es sich um Sonde (a), welche das N-terminale Polypeptid eines Inteins und das N-terminale Polypeptid eines markierten Proteins umfasst, und Sonde (b), welche das C-terminale Polypeptid des Inteins und das C-terminale Polypeptid des markierten Proteins umfasst, handelt.

2. Sonde zur Analyse von Protein-Protein-Wechselwirkung nach Anspruch 1, wobei der C-Terminus der Sonde (a) und der N-Terminus der Sonde (b) jeweils eine Linkersequenz enthalten.

3. Sonde zur Analyse von Protein-Protein-Wechselwirkung nach Anspruch 1 oder 2, wobei das Intein eine Endonuklease ist, die von Hefe-VMA abgeleitet ist.

4. Sonde zur Analyse von Protein-Protein-Wechselwirkung nach Anspruch 1 oder 2, wobei das Intein DnaE, erhalten aus Cyanobacterium, Ctr-VMA, erhalten aus Candida fungi, Mtu-recA, erhalten aus Mycobakterium tuberculosis, oder Tac-VMA, erhalten aus Thermoplasma acidophilum, ist.

5. Sonde zur Analyse von Protein-Protein-Wechselwirkung nach den Ansprüchen 1 bis 4, wobei das markierte Protein ein fluoreszierendes Protein ist.

6. Sonde zur Analyse von Protein-Protein-Wechselwirkung nach Anspruch 5, wobei das fluoreszierende Protein ein grün fluoreszierendes Protein ist.

7. Sonde zur Analyse von Protein-Protein-Wechselwirkung nach den Ansprüchen 1 bis 4, wobei das markierte Protein ein lumineszierendes Enzym ist.

8. Sonde zur Analyse von Protein-Protein-Wechselwirkung nach Anspruch 7, wobei das lumineszierende Enzym eine Luciferase ist.

9. Sonde nach Anspruch 8, wobei die Luciferase die folgenden Mutationen R437C, D436Y und I434A aufweist.

10. Polynukleotid, codierend für die Sonde nach irgendeinem der Ansprüche 1 bis 9.

11. Vektor, enthaltend das Polynukleotid nach Anspruch 10.

12. Eukaryotische Zelle, enthaltend das Polynukleotid nach Anspruch 10 oder den Vektor nach Anspruch 11.

13. Verfahren zur Analyse von Protein-Protein-Wechselwirkung, umfassend:
Veranlassung eines Proteins, das mit einer Sonde a wie in den Ansprüchen 1 bis 9 beschrieben verknüpft ist, und eines Proteins, das mit einer Sonde b wie in den Ansprüchen 1 bis 9 verknüpft ist, zur Koexistenz in einem System; und
Nachweis des Signals, das von dem markierten Protein emittiert wird.

14. Verfahren zur Analyse von Protein-Protein-Wechselwirkung nach Anspruch 13, wobei ein Polynukleotid, welches die Sonde nach irgendeinem der Ansprüche 1 bis 9 exprimiert, in eine eukaryotische Zelle eingeführt wird, wodurch das mit der Sonde a verknüpfte Protein und das mit der Sonde b verknüpfte Protein zur Koexistenz in der Zelle veranlasst werden.

## Revendications

1. Sonde pour l'analyse d'une interaction protéine-protéine entre deux protéines, consistant en deux sondes qui sont :
la sonde (a) qui comprend le polypeptide N-terminal d'une intéine et le polypeptide N-terminal d'une protéine marquée ; et la sonde (b) qui comprend le polypeptide C-terminal de l'intéine et le polypeptide C-terminal de la protéine marquée.

2. Sonde pour l'analyse d'une interaction protéine-protéine selon la revendication 1, dans laquelle l'extrémité C-terminale de la sonde (a) et l'extrémité N-terminale de la sonde (b) contiennent chacune une séquence de liaison.

3. Sonde pour l'analyse d'une interaction protéine-protéine selon la revendication 1 ou 2, dans laquelle l'intéine est une endonucléase dérivée de l'acide vanillyl-mandélique (VMA) de levure.

4. Sonde pour l'analyse d'une interaction protéine-protéine selon la revendication 1 ou 2, dans laquelle l'intéine est DnaE dérivé d'une cyanobactérie, Ctr VMA dérivé d'un champignon Candida, Mtu recA dérivé de la tuberculose d'une mycobactérie ou Tac VMA dérivé de Thermoplasma acidophilum.

5. Sonde pour l'analyse d'une interaction protéine-protéine selon les revendications 1 à 4, dans laquelle la protéine marquée est une protéine fluorescente.

6. Sonde pour l'analyse d'une interaction protéine-protéine selon la revendication 5, dans laquelle la protéine fluorescente est une protéine à fluorescence verte.

7. Sonde pour l'analyse d'une interaction protéine-protéine selon les revendications 1 à 4, dans laquelle la protéine marquée est une enzyme luminescente.

8. Sonde pour l'analyse d'une interaction protéine-protéine selon la revendication 7, dans laquelle l'enzyme luminescente est une luciférase.

9. Sonde selon la revendication 8, dans laquelle la luciférase a les mutations suivantes : R437C, D436Y et I434A.

10. Polynucléotide codant la sonde selon l'une quelconque des revendications 1 à 9.

11. Vecteur contenant le polynucléotide selon la revendication 10.

12. Cellule eucaryote contenant le polynucléotide selon la revendication 10 ou le vecteur selon la revendication 11.

13. Procédé d'analyse d'une interaction protéine-protéine comprenant :
le fait qu'une protéine liée à une sonde a tel que décrit dans les revendications 1 à 9 et qu'une protéine liée à une sonde b tel que décrit dans les revendications 1 à 9 coexistent dans un système ; et
la détection du signal émis par la protéine marquée.

14. Procédé pour une analyse d'une interaction protéine-protéine selon la revendication 13, dans lequel un polynucléotide qui exprime la sonde selon l'une quelconque des revendications 1 à 9 est introduit dans une cellule eucaryote, faisant ainsi en sorte que la protéine liée à la sonde a et la protéine liée à la sonde b coexistent dans la cellule.
